# EUROPEAN PATENT APPLICATION

(11) **EP 3 323 349 A1**
(43) Date of publication of application: **23.05.2018**
(21) Application number: 17201627.1
(22) Date of filing: 14.11.2017
(51) Int. Cl.: A61B 8/00, H05K 7/20

(54) **COOLING SYSTEM FOR ULTRASOUND DIAGNOSIS APPARATUS**

(30) Priority: 21.11.2016 KR 20160154971
(71) Applicant: Samsung Electronics Co., Ltd., Gyeonggi-do 16677 (KR)
(72) Inventor: YUN, Alexander, Seoul (KR); SHIN, Sok-jae, Yongin-si, Gyeonggi-do (KR); WOO, Ki-chul, Seoul (KR)
(74) Representative: Gulde & Partner

(57) **Abstract**

Provided is an ultrasound diagnosis apparatus cooling system including: a case configured to contain hardware components for operating an ultrasound diagnosis apparatus; a cooler located in a first direction with respect to the case and configured to cause air to flow in the first direction in the case; and an air distribution layer located between the case and the cooler and configured to direct air from the case to the cooler.

## Description

### CROSS-REFERENCE TO RELATED APPLICATIONS

This application claims priority from Korean Patent Application No. 10-2016-0154971, filed on November 21, 2016 in the Korean Intellectual Property Office, the disclosures of which are incorporated herein in their entireties by reference.

### BACKGROUND

### 1. Field

Apparatuses and methods consistent with exemplary embodiments relate to cooling systems in ultrasound diagnosis apparatuses.

### 2. Description of the Related Art

Ultrasound diagnosis apparatuses transmit ultrasound signals generated by transducers of a probe to an object and detect information about signals reflected from the object, thereby obtaining at least one image of an internal part, such as soft tissues or blood flow, of the object.

An ultrasound diagnosis apparatus generates heat therein during its operation. The ultrasound diagnosis apparatus may be cooled by a cooling system.

### SUMMARY

One or more exemplary embodiments may provide an ultrasound diagnosis apparatus cooling system including: a case configured to contain hardware components for operating an ultrasound diagnosis apparatus; a cooler located in a first direction with respect to the case and configured to cause air to flow in the first direction in the case; and an air distribution layer located between the case and the cooler and configured to direct the air from the case to the cooler.

According to an exemplary embodiment, the air distribution layer may include an air channel to direct the air flowing in the first direction from the case to the cooler.

According to an exemplary embodiment, the air channel may be a cylindrical channel.

According to an exemplary embodiment, the air channel may include an air inlet and an air outlet having different shapes from each other.

According to an exemplary embodiment, the air channel may include an air inlet facing toward an empty space of the case.

According to an exemplary embodiment, the air distribution layer may include a plurality of air channels to direct the air flowing in the first direction from the case to the cooler.

According to an exemplary embodiment, the plurality of air channels may together share one air outlet.

According to an exemplary embodiment, the plurality of air channels may have differently shaped air inlets.

According to an exemplary embodiment, the case may include a plurality of chambers containing the hardware components, and the plurality of air channels may respectively include air inlets facing toward the plurality of chambers respectively.

According to an exemplary embodiment, the plurality of air channels may respectively include air inlets facing toward the plurality of chambers respectively.

According to an exemplary embodiment, the plurality of air channels may respectively include air inlets, each air inlet of the inlets having a size proportional to an amount of heat generated in the respective chamber of the plurality of chambers to which the air inlet faces.

According to an exemplary embodiment, the cooler may include a fan located at a center of the cooler.

According to an exemplary embodiment, the air distribution layer may include an air channel, and the fan is in contact with an air outlet of the air channel.

According to an exemplary embodiment, the air outlet of the air channel may have a shape corresponding to a shape of the fan.

According to an exemplary embodiment, the ultrasound diagnosis apparatus cooling system may further include a cart on which the ultrasound diagnosis apparatus cooling system is mounted, wherein the cart comprises an air outlet corresponding to the fan.

According to an exemplary embodiment, the cooler may include a plurality of fans discharging air from the cooler in the first direction and a second direction perpendicular to the first direction.

According to an exemplary embodiment, the plurality of fans may be located along the second direction in the cooler.

According to an exemplary embodiment, the first direction may be an upward direction or downward direction.

According to an exemplary embodiment, the case may include an air inlet pulling air from outside of the case.

According to an exemplary embodiment, the air inlet may be located in at least one of a top side, a front side, a back side, a left side, and a right side of the case.

One or more exemplary embodiments may provide a cooling apparatus, including: a case configured to contain hardware components for operating an ultrasound diagnosis apparatus; a cooler, outside the case, including a fan to cause air to flow in the case; and an air distribution layer between the case and the cooler, and including an air channel to direct the air flowing in the case from the case to the cooler, wherein the case, the cooler and the air distribution layer are positioned with respect to each other so that, when the cooling apparatus is supported on a surface, the cooler is below the case, the fan causes the air to flow in a downward direction, with respect to the surface, in the case, and the fan is positioned directly below, or inside, an outlet of the air channel.

One or more exemplary embodiments may provide the surface on which the cooling apparatus may be supported is a floor or a surface of a rack.

### BRIEF DESCRIPTION OF THE DRAWINGS

These and/or other aspects will become apparent and more readily appreciated from the following description of the embodiments, taken in conjunction with the accompanying drawings in which:
FIG. 1 illustrates a perspective view of an ultrasound diagnosis apparatus cooling system according to an example embodiment.
FIG. 2 illustrates a cross-sectional view of an ultrasound diagnosis apparatus cooling system according to an example embodiment.
FIG. 3 illustrates a cross-sectional view of an ultrasound diagnosis apparatus cooling system and a top side of air distribution layers, according to an example embodiment.
FIG. 4 illustrates a cross-sectional view of an ultrasound diagnosis apparatus cooling system and a top side of an air distribution layer, according to an example embodiment.
FIG. 5 illustrates a cross-sectional view of an ultrasound diagnosis apparatus cooling system and a top side and a bottom side of an air distribution layer, according to an example embodiment.
FIG. 6 illustrates a cross-sectional view of an ultrasound diagnosis apparatus cooling system according to an example embodiment.
FIG. 7 illustrates a cross-sectional view of an ultrasound diagnosis apparatus cooling system according to an example embodiment.
FIG. 8 illustrates a perspective view of an ultrasound diagnosis apparatus cooling system and a top side of a case, according to an example embodiment.
FIG. 9 illustrates a top side of an air distribution layer according to an example embodiment.
FIG. 10 illustrates a top side and a bottom side of an air distribution layer according to an example embodiment.
FIG. 11 illustrates a top side of an air distribution layer according to an example embodiment.
FIG. 12 illustrates a cross-sectional view of an ultrasound diagnosis apparatus cooling system according to an example embodiment.
FIG. 13 illustrates a perspective view of an ultrasound diagnosis apparatus cooling system according to an example embodiment.
FIG. 14 illustrates a perspective view of an ultrasound diagnosis cooling system according to an example embodiment.
FIG. 15 is a block diagram illustrating an ultrasound diagnosis apparatus according to an exemplary embodiment.
FIGS. 16A, 16B, and 16C are diagrams respectively illustrating an ultrasound diagnosis apparatus according to an exemplary embodiment

### DETAILED DESCRIPTION

In the following description, the same reference numerals are used for the same elements even when referring to different drawings. The matters defined in the description, such as detailed construction and elements, are provided to assist in a comprehensive understanding of exemplary embodiments.

Thus, it will be apparent that exemplary embodiments can be carried out without those specifically defined matters. Also, well-known functions or constructions are not described in detail since they would obscure exemplary embodiments with unnecessary detail. Terms such as "part" and "portion" used herein denote those that may be embodied by software or hardware. According to exemplary embodiments, a plurality of parts or portions may be embodied by a single unit or element, or a single part or portion may include a plurality of elements. Various exemplary embodiments will be explained by referring to the accompanying drawings.

In exemplary embodiments, an image may include any medical image acquired by various medical imaging apparatuses such as a magnetic resonance imaging (MRI) apparatus, a computed tomography (CT) apparatus, an ultrasound imaging apparatus, or an X-ray apparatus.

Also, in the present specification, an "object", which is a thing to be imaged, may include a human, an animal, or a part thereof. For example, an object may include a part of a human, that is, an organ or a tissue, or a phantom.

Throughout the specification, an ultrasound image refers to an image of an object, which is processed based on ultrasound signals transmitted to the object and reflected therefrom.

Reference will now be made in detail to embodiments, examples of which are illustrated in the accompanying drawings.

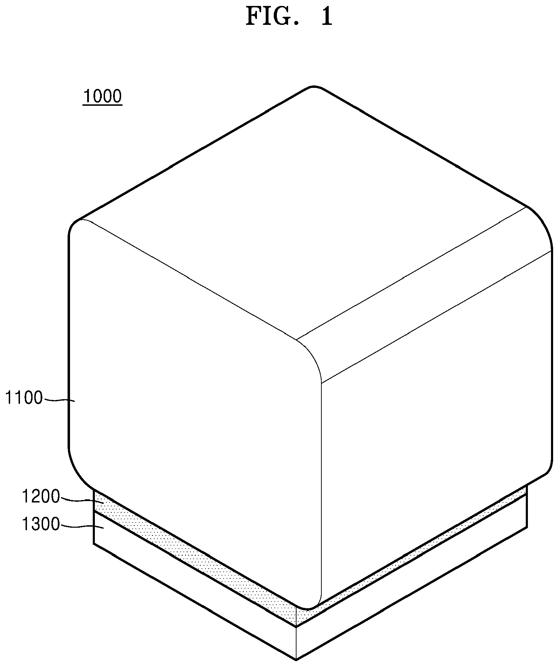

FIG. 1 illustrates a perspective view of an ultrasound diagnosis apparatus cooling system 1000 according to an example embodiment.

The ultrasound diagnosis apparatus cooling system 1000 may include a case 1100, a cooler 1300, and an air distribution layer 1200.

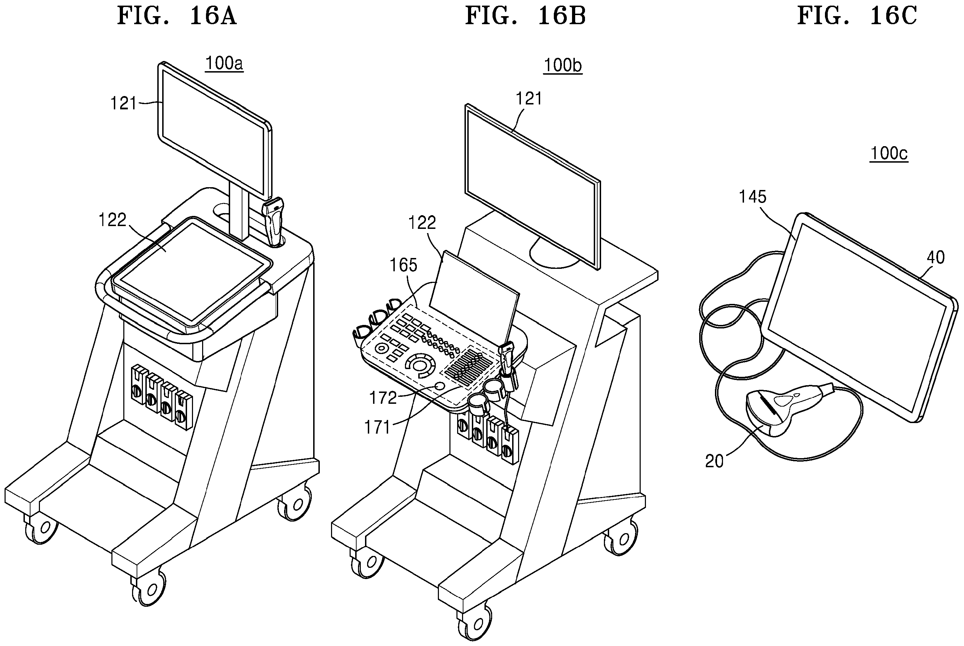

The case 1100 may contain hardware components for operating an ultrasound diagnosis apparatus. The hardware components may include a processing unit, a power supply, and an application circuit, but are not limited thereto. For example, the hardware components may be among elements of an ultrasound diagnosis apparatus illustrated in FIGS. 16A, 16B, and 16C.

The cooler 1300 may be located in a first direction with respect to the case 1100 to cause air to flow in the first direction in order to dissipate heat from the case 1100. The first direction in FIG. 1 is a downward direction, but is not limited to the downward direction. As should be understood from FIG. 1, the downward direction is with respect to a supporting surface, such as a floor or surface of a rack, on which the ultrasound diagnosis apparatus cooling system is supported. The cooler 1300 is illustrated as being separate from the air distribution layer 1200 in FIG. 1, but may be a part of the air distribution layer 1200.

The cooler 1300 may let heat generated in the case 1100 out of the case 1100. The hardware components may malfunction if heat, generated by the hardware components in the case 1100, is not cooled.

A conventional ultrasound diagnosis apparatus includes an inlet fan for pulling air into it, and an exhaust fan for exhausting air in order to reduce heat generated in its case. The inlet fan and the exhaust fan may be mounted or located on the case of the ultrasound diagnosis apparatus. As the number of fans increases, the cooling effect improves, but noise also increases.

Locations of fans may be changed to improve air circulation. For example, air circulation may improve when using an inlet fan and an exhaust fan facing each other by, for example, locating them on a top side and a bottom side of the ultrasound diagnosis apparatus respectively.

However, turbulence may be generated by the inlet and exhaust fans. Turbulence interrupts air circulation and increases noise. When the inlet fan and the exhaust fan are located in a bottom side and a back side of the ultrasound diagnosis apparatus respectively, air moving upward due to the inlet fan located in the bottom side of the case is bent toward the back side of the case. That is, air flow is bent during its movement, which may cause turbulence.

In an example embodiment, the air distribution layer 1200 may be located between the case 1100 and the cooler 1300, and direct air from the case 1100 to the cooler. Thus, turbulence may be reduced while improving cooling efficiency and reducing noise.

The air distribution layer 1200 may be embodied by plastic, aluminum, or stainless steel, but is not limited thereto. When an ultrasound diagnosis apparatus is located on the air distribution layer 1200, the air distribution layer 1200 may be embodied by an appropriate material to support the ultrasound diagnosis apparatus. The air distribution layer 1200 may have an appropriate thickness to support the ultrasound diagnosis apparatus and to improve air circulation.

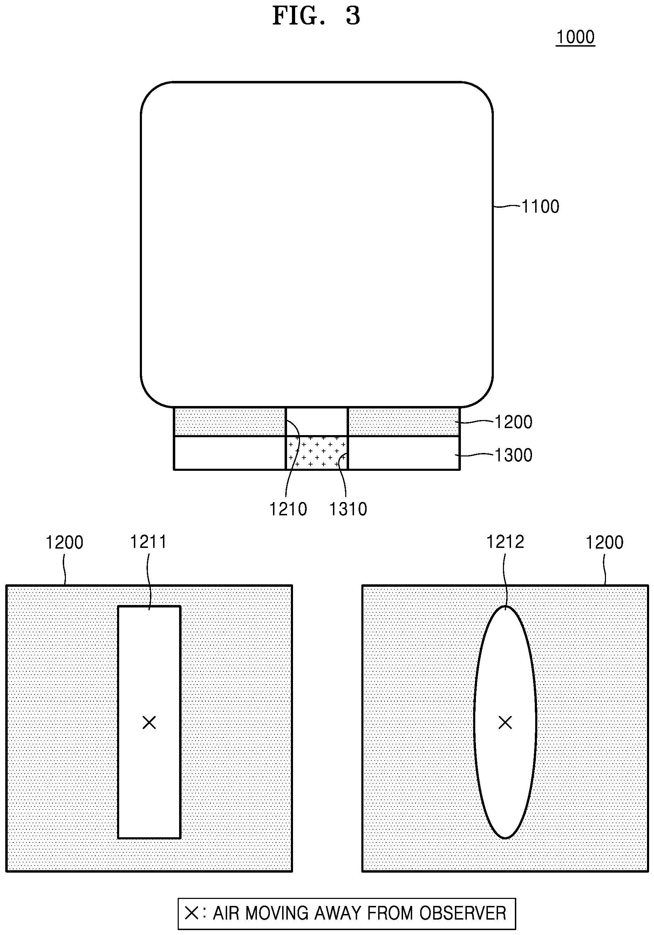

Various shapes of the air distribution layer 1200 will be explained later below by referring to FIGS. 3, 4, 5, 6, 7, 8, 9, 10, and 11.

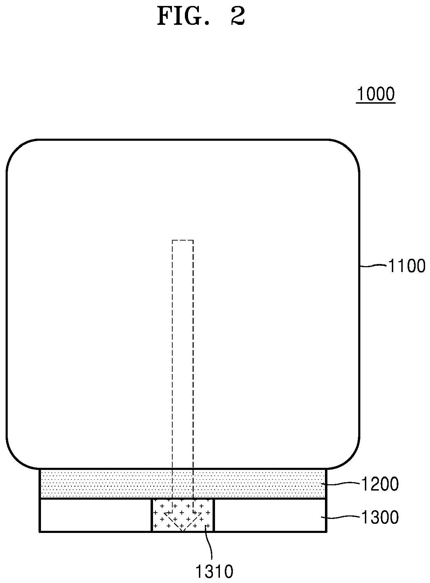

FIG. 2 illustrates a cross-sectional view of an ultrasound diagnosis apparatus cooling system 1000 according to an example embodiment.

As illustrated in FIG. 2, the cooler 1300 may include a fan 1310 according to an example embodiment. The fan 1310 may cause air to flow downward in the case 1100, and exhaust air to the outside of the cooler 1300. For example, air may be exhausted to sides of the cooler 1300, but is not limited thereto, and may be exhausted under the cooler 1300. The fan 1310 may be located between a top side and a bottom side of the cooler 1300 as illustrated in FIG. 2, but is not limited thereto. The fan 1310 may be located in the top side or the bottom side of the cooler 1300.

The case 1100 may include hardware components, and turbulence may be generated by air flowing, due to the fan 1310, through the hardware components. In an example embodiment, an ultrasound diagnosis apparatus cooling system 1000 causes air to flow in a first direction in the case 1100 containing the hardware components, and turbulence caused by air flow being bent may be reduced.

In an exemplary embodiment, the fan 1310 may be located at the center of the cooler 1300, but is not limited thereto. For example, the fan 1310 may be located at sides of the cooler 1300, or located at a place other than the center.

The fan 1310 is illustrated as being in contact with the air distribution layer 1200 in FIG. 2, but the fan 1310 may be separated from the air distribution layer 1200. The fan 1310 and the air distribution layer 1200 may be in direct contact, but may be in indirect contact via a medium such as a tube. An exhaust effect may improve by contacting the fan 1310 with the air distribution layer 1200.

FIG. 3 illustrates a cross-sectional view of an ultrasound diagnosis apparatus cooling system 1000 and a top side of air distribution layers 1200, according to an example embodiment.

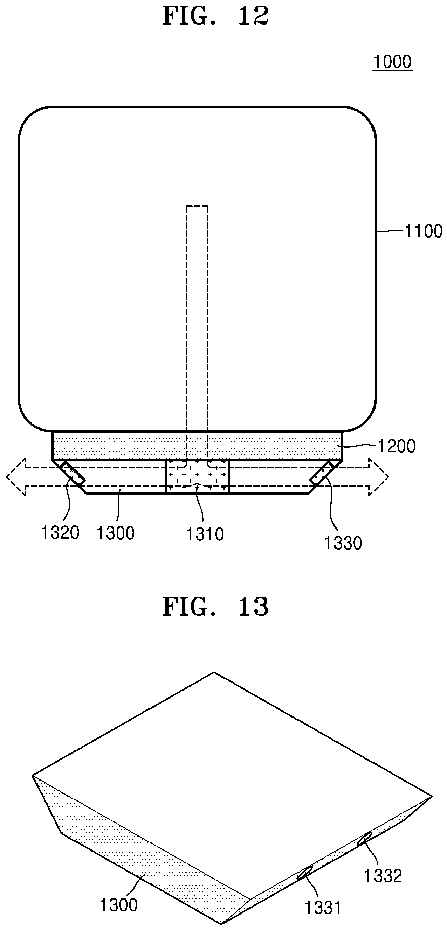

As illustrated in FIG. 3, the air distribution layer 1200 may include an air channel 1210 according to an example embodiment. The air channel 1210 may be an empty space in the air distribution layer 1200, but is not limited thereto. Due to a fan 1310 located under the air distribution layer 1200, hot air moves downward in the case 1100, and arrives at the cooler 1300 via the air channel 1210. Air passing the air channel 1210 may be exhausted to the outside through the fan 1310. The fan 1310 may be located between a top side and a bottom side of the cooler 1300 as illustrated in FIG. 3, but is not limited thereto. The fan 1310 may be located in sides of the cooler 1300 as illustrated in FIG. 12.

The air channel may have various shapes. For example, the air channel 1210 may have a rectangular air inlet 1211. In an example embodiment, the air channel 1210 may have an air outlet having the same shape as the air inlet 1211. Accordingly, the air channel 1210 may have a shape of a rectangular pillar.

In an example embodiment, the air channel 1210 may have an air outlet having a similar shape to the air inlet 1211 with the same ratios, but different dimensions. Accordingly, the air channel 1210 may have a shape of a truncated pyramid or a frustum of a square pyramid.

The air channel 1210 may have an oval-shaped air inlet as illustrated in FIG. 3. In an example embodiment, the air channel 1210 may have an air outlet having the same shape as the air inlet 1212, and thus, have a shape of a cylinder accordingly. In an example embodiment, the air channel 1210 may have an air outlet having a similar shape to the air inlet 1211, with the same ratios, but a different size. Accordingly, the air channel 1210 may have a shape of a truncated cone.

The fan 1310 may be in contact with the air outlet of the air channel 1210. The fan 1310 and the air outlet of the air channel 1210 may be in direct contact, but be in indirect contact through a medium such as a tube. Furthermore, the fan 1310 may be located inside of the air outlet of the air channel 1210. An exhaust effect may be improved by contacting the fan 1310 with the air outlet of the air distribution layer 1210. The fan 1310 is illustrated as being in contact with the air channel 1210 in FIG. 3, but the fan 1310 may be separated from the air channel 1210.

In an example embodiment, the ultrasound diagnosis apparatus cooling system 1000 may further include a cart on which the ultrasound diagnosis apparatus cooling system 1000 is mounted. The cart may include an air outlet whose size and location may correspond to the fan 1310 located in the cooler 1300. For example, the air outlet of the cart may be located in a direction in which air is exhausted by the fan 1310.

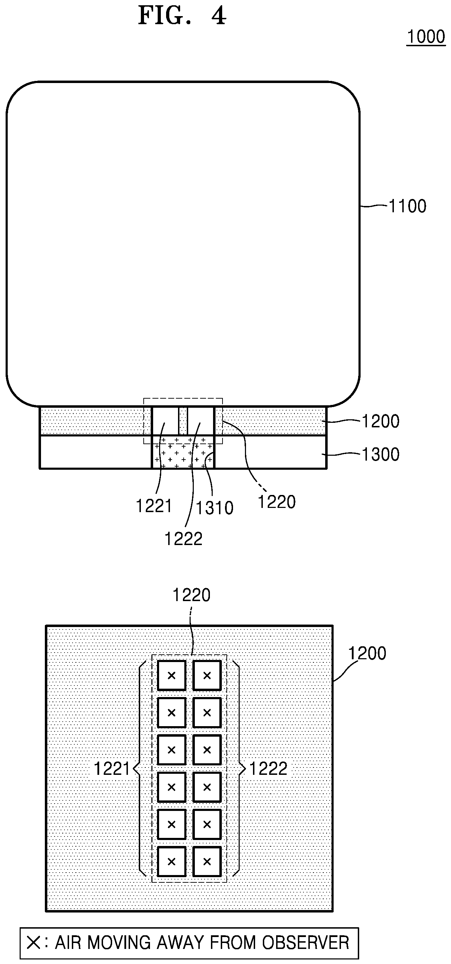

FIG. 4 illustrates a cross-sectional view of an ultrasound diagnosis apparatus cooling system 1000 and a top side of an air distribution layer 1200, according to an example embodiment.

As illustrated in FIG. 4, the air distribution layer 1200 may include an air channel 1220 including a plurality of air channel bundles 1221 and 1222, according to an example embodiment. An amount of air being pulled from the case 1100 may depend on a density of the air channel bundles 1221 and 1222 in the air channel 1220. Therefore, an air channel that has air channel bundles of higher density (having more empty space in the air channel) may be located under a region where more heat is generated than other regions in the case 1100.

The air channel bundles 1221 and 1222 may have various shapes. In one embodiment, air inlets and air outlets of the air channel bundles 1221 and 1222 may have various shapes.

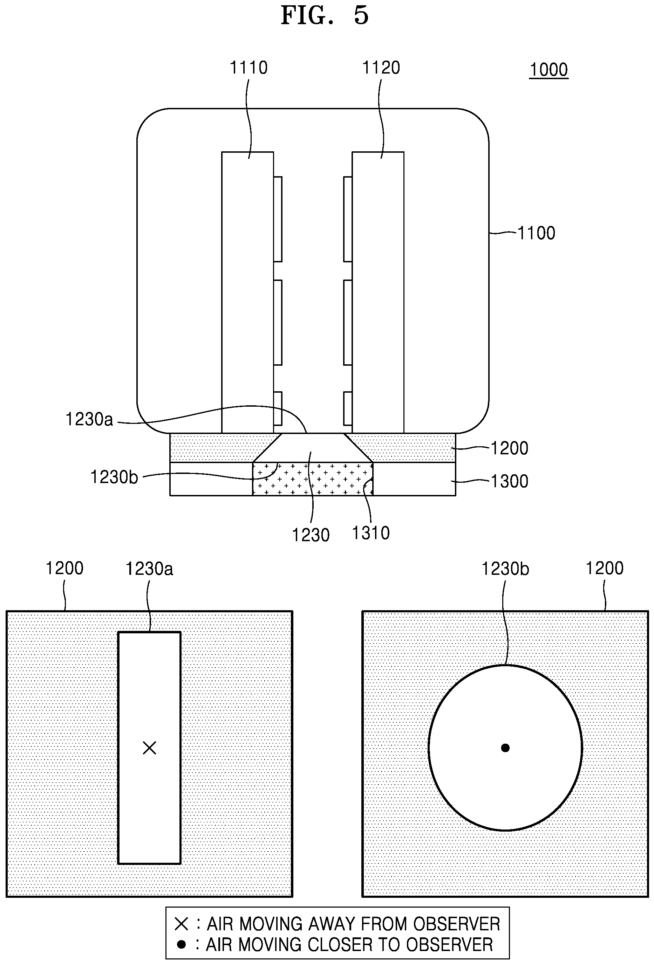

FIG. 5 illustrates a cross-sectional view of an ultrasound diagnosis apparatus cooling system 1000 and a top side and a bottom side of an air distribution layer 1200, according to an example embodiment.

As illustrated in FIG. 5, a case 1100 of the ultrasound diagnosis apparatus may include components 1110 and 1120 for operating the ultrasound diagnosis apparatus according to an example embodiment. The components 1110 and 1120 may have shapes of a plate, and may stand in the case 1100.

In an example embodiment, a location of an air inlet 1230a of an air channel 1230 may correspond to regions where the components 1110 and 1120 are not located in the case 1100. For example, the air inlet 1230a may face toward a region which is not occupied by the components 1110 and 1120 in a bottom side of the case 1100. When the air inlet 1230a faces toward an empty region in the bottom side of the case 1100, hot air may arrive at the air inlet 1230a efficiently. In an example embodiment, the air inlet 1230a may face toward a space which is not occupied by the components 1110 and 1120 in the case 1100.

In an example embodiment, the air inlet 1230a and an air outlet 1230b of the air channel 1230 may have different shapes as illustrated in FIG. 5. For example, the air inlet 1230a may have a rectangular shape, and the air outlet 1230b may have a circular shape. In an example embodiment, the air outlet 1230b and the air inlet 1230a may appear similar to each other with the same ratios, but have different sizes.

In an example embodiment, a shape of the air outlet 1230b may correspond to a fan that is located under the air outlet 1230b. For example, the air outlet 1230b may have the same shape as the outer edge of the fan that is located under the air outlet 1230b. Therefore, the fan may cause air to flow in the case 1100 efficiently.

In an example embodiment, a shape of the air outlet 1230b may correspond to an empty space where hardware components are not located in the case 1100. For example, the air outlet 1230a may have the same shape as a region which is not occupied by hardware components in a bottom side of the case 1100. Therefore, hot air may move downward in the case by the air inlet 1230a efficiently.

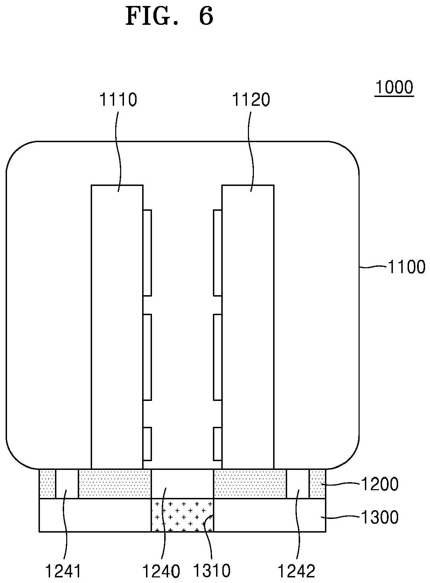

FIG. 6 illustrates a cross-sectional view of an ultrasound diagnosis apparatus cooling system 1000 according to an example embodiment.

As illustrated in FIG. 6, an air distribution layer 1200 may include a plurality of air channels 1240, 1241, and 1242 according to an example embodiment.

In an example embodiment, the air channels 1240, 1241, and 1242 may respectively face toward empty spaces in the case 1100. Therefore, hot air may flow downward in the case 1100 via the air channels 1240, 1241, and 1242.

A fan 1310 is illustrated as being located under the air channel 1240 in FIG. 6, but a plurality of fans may be located under the plurality of air channels respectively. Accordingly, cooling efficiency may increase.

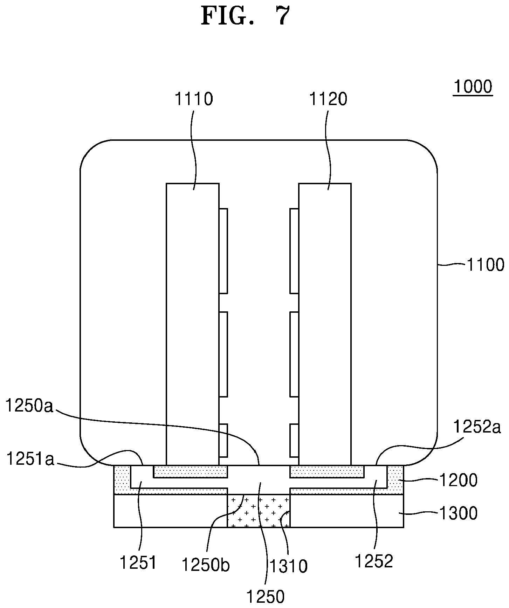

FIG. 7 illustrates a cross-sectional view of an ultrasound diagnosis apparatus cooling system 1000 according to an example embodiment.

As illustrated in FIG. 7, air channels 1250, 1251, and 1252 may share one air outlet 1250b according to an example embodiment.

Therefore, hot air may flow downward in the case 1100 via the air channels more efficiently in comparison with an embodiment illustrated in FIG. 6, even when using the same fan 1310.

The air channel bundles may share at least one air outlet.

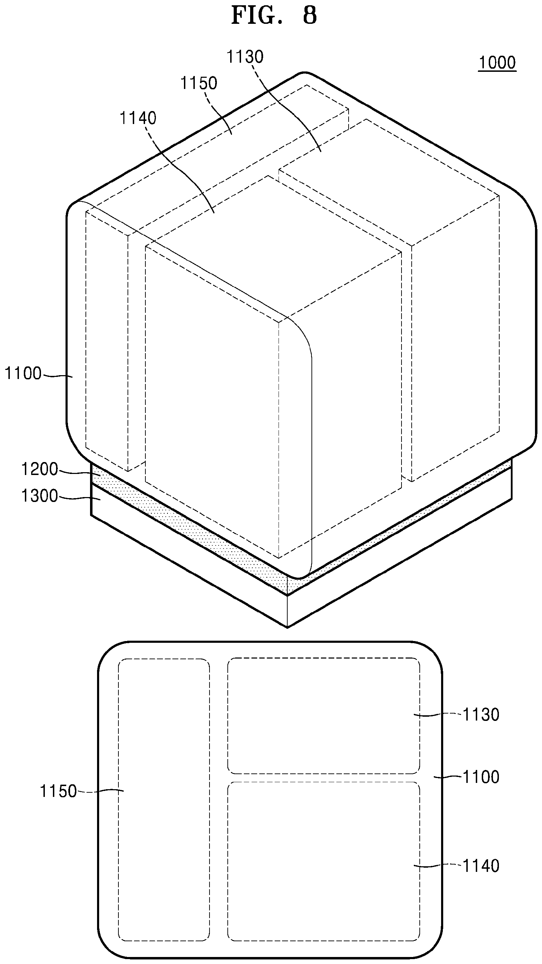

FIG. 8 illustrates a perspective view of an ultrasound diagnosis apparatus cooling system 1000 and a top side of a case 1100 according to an example embodiment.

As illustrated in FIG. 8, a case 1100 may include a plurality of chambers 1130, 1140, and 1150, according to an example embodiment.

The chambers 1130, 1140, and 1150 may contain hardware components for operating an ultrasound diagnosis apparatus. For example, the chamber 1130 may include a processing unit, and the chamber 1140 may include an application circuit, and the chamber 1150 may include a power supply, but are not limited thereto. Locations of chambers, the number of chambers, and hardware components contained in chambers may vary according to embodiments.

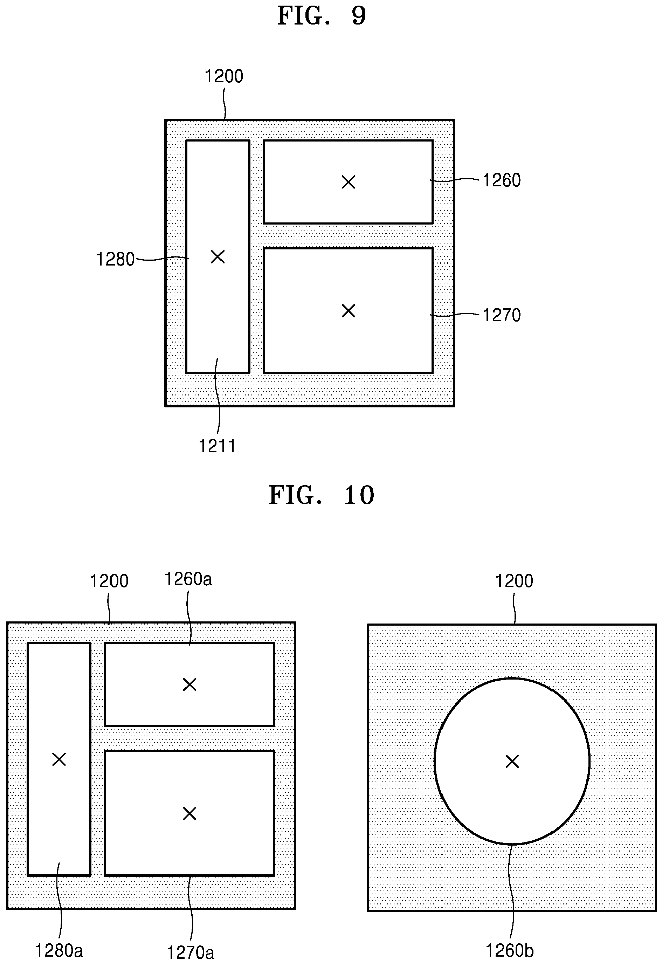

FIG. 9 illustrates a top side of an air distribution layer 1200 according to an example embodiment.

As illustrated in FIG. 9, an air distribution layer 1200 may include a plurality of air channels 1260, 1270, and 1280, according to an example embodiment.

The plurality of air channels 1260, 1270, and 1280 may respectively correspond to the plurality of chambers 1130, 1140, and 1150. For example, air inlets of the air channels 1260, 1270, and 1280 may face toward the chambers 1130, 1140, and 1150 respectively. Therefore, hot air in the chambers 1130, 1140, and 1150 in the case may move downward efficiently via the air channels 1260, 1270, and 1280 respectively.

In an example embodiment, the size of each air inlet of the air channels 1260, 1270, 1280 may be proportional to an area occupied by each of the chambers 1130, 1140, and 1150 in a bottom side of the case 1100.

In an example embodiment, the size of each air inlet of the air channels 1260, 1270, 1280 may be proportional to an amount of heat generated in each of the chambers 1130, 1140, and 1150. An air inlet corresponding to a chamber relatively generating more heat may have a bigger size than other air inlets corresponding to chambers generating relatively less heat. Air channels 1260, 1270, and 1280 may share one air outlet 1260b as illustrated in FIG. 10, and more amount of air may be pulled from a chamber generating relatively more heat than other chambers, thereby, cooling an ultrasound diagnosis apparatus more efficiently.

FIG. 10 illustrates a top side and a bottom side of an air distribution layer 1200 according to an example embodiment.

The air channels 1260, 1270, and 1280 of FIG. 9 may share one air outlet 1260b as illustrated in FIG. 10. The air outlet 1260b may have a different shape to air inlets 1260a, 1270a, and 1280a. For example, the air inlets 1260, 1270a, and 1280a may have rectangular shapes, and the air outlet 1260b may have a circular shape, but are not limited thereto.

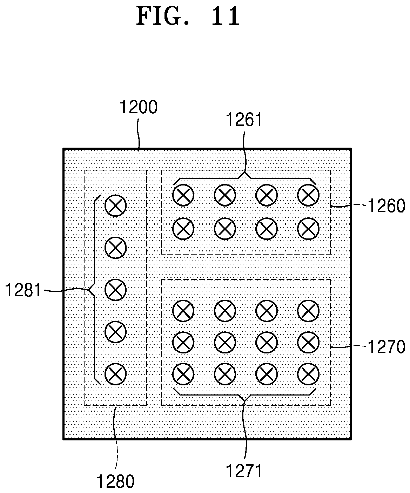

FIG. 11 illustrates a top side of an air distribution layer 1200 according to an example embodiment.

The air channels 1260, 1270, and 1280 of FIG. 9 may respectively include a plurality of air channel bundles 1261, 1271, and 1271 as illustrated in FIG. 11.

FIG. 12 illustrates a cross-sectional view of an ultrasound diagnosis apparatus cooling system 1000 according to an example embodiment.

As illustrated in FIG. 12, a cooler 1300 may include fans 1320 and 1330 which are located in both sides of the cooler 1300 to exhaust air directed via an air distribution layer 1200 to the outside. The fans 1320 and 1330 may be referred to as side fans.

The side fans 1320 and 1330 may bend air flow in the cooler 1300 to sides of the cooler 1300. Air flow is bent in the cooler 1300, but not in the case 1100 containing hardware components, and thus, turbulence and noise may be reduced.

A fan 1310 and side fans 1320 and 1330 may be exhaust fans that operate together. Air exhausted from the case 1100 to the cooler 1300 by the fan 1310 may be exhausted to the outside by the side fans 1320 and 1330 efficiently.

The side fans 1320 and 1330 may lean at a certain degree as illustrated in FIG. 12 to pull air from the air distribution layer 1200 efficiently.

Two side fans 1320 and 1330 may be located along a virtual line crossing the sides of the cooler 1300 at the shortest distance, but are not limited thereto.

FIG. 13 illustrates a perspective view of an ultrasound diagnosis apparatus cooling system 1000 according to an example embodiment.

As illustrated in FIG. 13, two fans 1331 and 1332 may be located in a side of a cooler 1300.

The two fans 1331 and 1332 are illustrated as being located in the side of the cooler 1300 in FIG. 13, but more side fans may be located in the side and/or other sides.

In an example embodiment, a fan may be located in a front side and a back side of the cooler 1300.

In an example embodiment, the cooler 1300 may be detached from the case 1100. Therefore, the cooler 1300 may be maintained and cleaned conveniently.

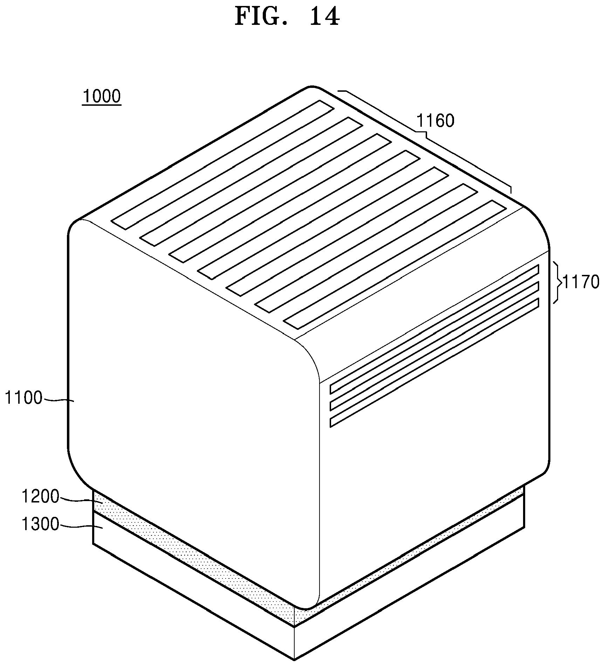

FIG. 14 illustrates a perspective view of an ultrasound diagnosis cooling system 1000 according to an example embodiment.

As illustrated in FIG. 14, a case 1100 of the ultrasound diagnosis cooling system 1000 may include air inlets 1160 and 1170 to bring air therein. The air inlets 1160 and 1170 may be located in a top side and a right side of the case 1100 as illustrated in FIG. 14, but are not limited thereto. For example, air inlets may be further located in a front side and a back side of the case 1100, and air inlets may be located in lateral sides only for allowing other components to be located on the case 1100.

While hot air in the case 1100 is exhausted from the case via the air distribution layer 1200 by the cooler 1300, cool air may flow into the case via air inlets of the case 1100 from the outside.

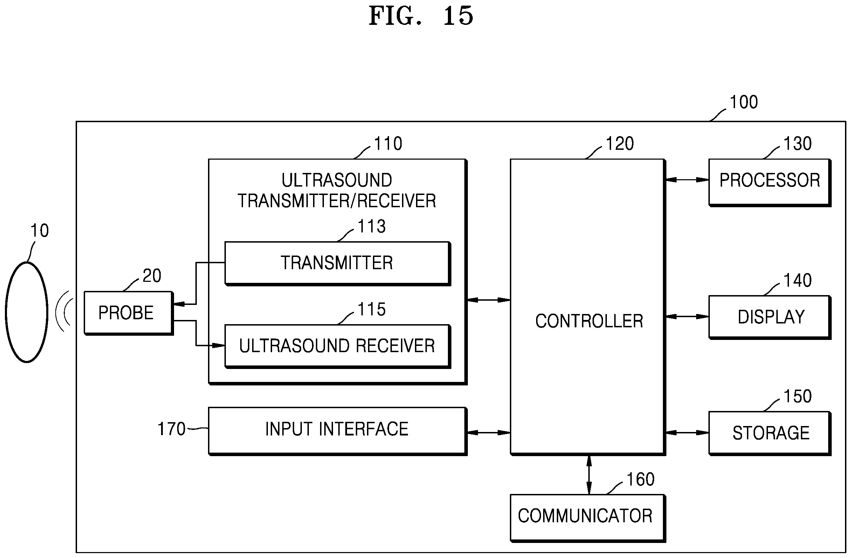

FIG. 15 is a block diagram illustrating a configuration of an ultrasound diagnosis apparatus 100, i.e., a diagnostic apparatus, according to an exemplary embodiment. Referring to FIG. 1, the ultrasound diagnosis apparatus 100 may include a probe 20, an ultrasound transceiver 110, a controller 120, an image processor 130, one or more displays 140, a storage 150, e.g., a memory, a communicator 160, i.e., a communication device or an interface, and an input interface 170.

The ultrasound diagnosis apparatus 100 may be a cart-type or a portable-type ultrasound diagnosis apparatus that is portable, moveable, mobile, or hand-held. Examples of the portable-type ultrasound diagnosis apparatus 100 may include a smart phone, a laptop computer, a personal digital assistant (PDA), and a tablet personal computer (PC), each of which may include a probe and a software application, but embodiments are not limited thereto.

The probe 20 may include a plurality of transducers. The plurality of transducers may transmit ultrasound signals to an object 10 in response to transmitting signals received by the probe 20, from a transmitter 113. The plurality of transducers may receive ultrasound signals reflected from the object 10 to generate reception signals. In addition, the probe 20 and the ultrasound diagnosis apparatus 100 may be formed in one body (e.g., disposed in a single housing), or the probe 20 and the ultrasound diagnosis apparatus 100 may be formed separately (e.g., disposed separately in separate housings) but linked by wire or wirelessly. In addition, the ultrasound diagnosis apparatus 100 may include one or more probes 20 according to embodiments.

The controller 120 may control the transmitter 113 to generate transmitting signals to be applied to each of the plurality of transducers based on a position and a focal point of the plurality of transducers included in the probe 20.

The controller 120 may control the ultrasound receiver 115 to generate ultrasound data by converting reception signals received from the probe 20 from analog to digital signals and summing the reception signals converted into digital form, based on a position and a focal point of the plurality of transducers.

The image processor 130 may generate an ultrasound image by using ultrasound data generated from the ultrasound receiver 115.

The display 140 may display a generated ultrasound image and various pieces of information processed by the ultrasound diagnosis apparatus 100. The ultrasound diagnosis apparatus 100 may include two or more displays 140 according to the present exemplary embodiment. The display 140 may include a touch screen in combination with a touch panel.

The controller 120 may control the operations of the ultrasound diagnosis apparatus 100 and flow of signals between the internal elements of the ultrasound diagnosis apparatus 100. The controller 120 may include a memory for storing a program or data to perform functions of the ultrasound diagnosis apparatus 100 and a processor and/or a microprocessor (not shown) for processing the program or data. For example, the controller 120 may control the operation of the ultrasound diagnosis apparatus 100 by receiving a control signal from the input interface 170 or an external apparatus.

The ultrasound diagnosis apparatus 100 may include the communicator 160 and may be connected to external apparatuses, for example, servers, medical apparatuses, and portable devices such as smart phones, tablet personal computers (PCs), wearable devices, etc., via the communicator 160.

The communicator 160 may include at least one element capable of communicating with the external apparatuses. For example, the communicator 160 may include at least one among a short-range communication module, a wired communication module, and a wireless communication module.

The communicator 160 may receive a control signal and data from an external apparatus and transmit the received control signal to the controller 120 so that the controller 120 may control the ultrasound diagnosis apparatus 100 in response to the received control signal.

The controller 120 may transmit a control signal to the external apparatus via the communicator 160 so that the external apparatus may be controlled in response to the control signal of the controller 120.

For example, the external apparatus connected to the ultrasound diagnosis apparatus 100 may process the data of the external apparatus in response to the control signal of the controller 120 received via the communicator 160.

A program for controlling the ultrasound diagnosis apparatus 100 may be installed in the external apparatus. The program may include command languages to perform some operations of the controller 120 or all operations of the controller 120.

The program may be pre-installed in the external apparatus or may be installed by a user of the external apparatus by downloading the program from a server that provides applications. The server that provides applications may include a recording medium where the program is stored.

The storage 150 may store various data or programs for driving and controlling the ultrasound diagnosis apparatus 100, input and/or output ultrasound data, ultrasound images, applications, etc.

The input interface 170 may receive a user's input to control the ultrasound diagnosis apparatus 100 and may include a keyboard, button, keypad, mouse, trackball, jog switch, knob, a touchpad, a touch screen, a microphone, a motion input means, a biometrics input means, etc. For example, the user's input may include inputs for manipulating buttons, keypads, mice, trackballs, jog switches, or knobs, inputs for touching a touchpad or a touch screen, a voice input, a motion input, and a bioinformation input, for example, iris recognition or fingerprint recognition, but an exemplary embodiment is not limited thereto.

An example of the ultrasound diagnosis apparatus 100 according to the present exemplary embodiment is described below with reference to FIGS. 16A, 16B, and 16C.

FIGS. 16A, 16B, and 16C are diagrams illustrating ultrasound diagnosis apparatus according to an exemplary embodiment.

Referring to FIGS. 16A and 16B, the ultrasound diagnosis apparatus 100 may include a main display 121 and a sub-display 122. At least one of the main display 121 and the sub-display 122 may include a touch screen. The main display 121 and the sub-display 122 may display ultrasound images and/or various information processed by the ultrasound diagnosis apparatus 100. The main display 121 and the sub-display 122 may provide graphical user interfaces (GUI), thereby receiving a user's inputs of data to control the ultrasound diagnosis apparatus 100. For example, the main display 121 may display an ultrasound image and the sub-display 122 may display a control panel to control display of the ultrasound image as a GUI. The sub-display 122 may receive an input of data to control the display of an image through the control panel displayed as a GUI. The ultrasound diagnosis apparatus 100 may control the display of the ultrasound image on the main display 121 by using the input control data.

Referring to FIG. 16B, the ultrasound diagnosis apparatus 100 may include a control panel 165. The control panel 165 may include buttons, trackballs, jog switches, or knobs, and may receive data to control the ultrasound diagnosis apparatus 100 from the user. For example, the control panel 165 may include a time gain compensation (TGC) button 171 and a freeze button 172. The TGC button 171 is to set a TGC value for each depth of an ultrasound image. Also, when an input of the freeze button 172 is detected during scanning of an ultrasound image, the ultrasound diagnosis apparatus 100 may keep displaying a frame image at that time point.

The buttons, trackballs, jog switches, and knobs included in the control panel 165 may be provided as a GUI to the main display 121 or the sub-display 122.

Referring to FIG. 16C, the ultrasound diagnosis apparatus 100 may include a portable device. An example of the portable ultrasound diagnosis apparatus 100 may include, for example, smart phones including probes and applications, laptop computers, personal digital assistants (PDAs), or tablet PCs, but an exemplary embodiment is not limited thereto.

The ultrasound diagnosis apparatus 100 may include the probe 20 and a main body 40. The probe 20 may be connected to one side of the main body 40 by wire or wirelessly. The main body 40 may include a touch screen 145. The touch screen 145 may display an ultrasound image, various pieces of information processed by the ultrasound diagnosis apparatus 100, and a GUI.

## Claims

1. An ultrasound diagnosis apparatus cooling system comprising:
a case configured to contain hardware components for operating an ultrasound diagnosis apparatus;
a cooler located outside the case and configured to cause air to flow in a first direction in the case; and
an air distribution layer located outside the case, between the case and the cooler, and configured to direct the air flowing in the first direction from the case to the cooler.

2. The ultrasound diagnosis apparatus cooling system of claim 1, wherein the air distribution layer comprises an air channel to direct the air flowing in the first direction from the case to the cooler.

3. The ultrasound diagnosis apparatus cooling system of claim 2, wherein the air channel is a cylindrical channel.

4. The ultrasound diagnosis apparatus cooling system of claim 2, wherein the air channel comprises an air inlet and an air outlet having different shapes from each other.

5. The ultrasound diagnosis apparatus cooling system of claim 2, wherein the air channel comprises an air inlet facing toward an empty space of the case.

6. The ultrasound diagnosis apparatus cooling system of claim 1, wherein the air distribution layer comprises a plurality of air channels to direct the air flowing in the first direction from the case to the cooler.

7. The ultrasound diagnosis apparatus cooling system of claim 6, wherein the plurality of air channels together share one air outlet.

8. The ultrasound diagnosis apparatus cooling system of claim 6, wherein the plurality of air channels have differently shaped air inlets.

9. The ultrasound diagnosis apparatus cooling system of claim 6, wherein the case comprises a plurality of chambers containing the hardware components, and the plurality of air channels respectively comprise air inlets facing toward the plurality of chambers respectively.

10. The ultrasound diagnosis apparatus cooling system of claim 9, wherein the plurality of air channels respectively comprise air inlets, each air inlet of the inlets having a size proportional to an amount of heat generated in the respective chamber of the plurality of chambers to which the air inlet faces.

11. The ultrasound diagnosis apparatus cooling system of claim 1, wherein the cooler comprises a fan located at a center of the cooler.

12. The ultrasound diagnosis apparatus cooling system of claim 11, wherein the air distribution layer comprises an air channel, and the fan is in contact with an air outlet of the air channel.

13. The ultrasound diagnosis apparatus cooling system of claim 12, wherein the air outlet of the air channel has a shape corresponding to a shape of the fan.

14. The ultrasound diagnosis apparatus cooling system of claim 1, wherein the cooler comprises a plurality of fans discharging air from the cooler in the first direction and a second direction perpendicular to the first direction.

15. The ultrasound diagnosis apparatus cooling system of claim 14, wherein the plurality of fans are located along the second direction in the cooler.
